# FASCICULE DE BREVET EUROPEEN

(11) **EP 0 852 569 B1**
(45) Date de publication et mention de la délivrance du brevet: **05.06.2002**
(21) Numéro de dépôt: 96932662.8
(22) Date de dépôt: 27.09.1996
(51) Int. Cl.: C01G 49/02

(54) **PROCEDE DE PREPARATION DE CATALYSEURS D'AMMOXYDATION**
VERFAHREN ZUR HERSTELLUNG VON AMMOXIDATIONSKATALYSATOREN
METHOD FOR PREPARING AMMOXIDATION CATALYSTS

(30) Priorité: 29.09.1995 FR 9511680
(43) Date de publication de la demande: 15.07.1998
(73) Titulaire: RHODIA POLYAMIDE INTERMEDIATES, 69190 Saint-Fons (FR)
(72) Inventeur: ALBONETTI, Stéfania, I-40026 Imola (IT); BLANCHARD, Gilbert, F-60330 Le Plessis-Belleville (FR); BURATTIN, Paolo, F-69002 Lyon (FR); CAVANI, Fabrizio, I-41100 Modena (IT); TRIFIRO, Ferruccio, I-40136 Bologna (IT)
(74) Mandataire: Esson, Jean-Pierre
(86) Numéro de dépôt international: FR9601510
(87) Numéro de publication internationale: WO9712839

(56) Documents cités:
- EP-A- 0 306 973
- EP-A- 0 455 529
- EP-A- 0 558 424
- US-A- 4 060 500
- US-A- 4 257 921
- US-A- 5 008 427

## Description

La présente invention concerne un nouveau procédé de préparation d'oxydes mixtes de vanadium, d'antimoine et de fer, ainsi que l'utilisation de ces oxydes comme catalyseurs pour l'ammoxydation des alcanes.

Certains oxydes mixtes de vanadium et d'antimoine ou de vanadium, d'antimoine et d'autres métaux sont des composés connus qui ont été décrits, parmi de nombreux autres oxydes mixtes, dans le brevet FR-A-2 072 334.

Dans le brevet US 5 008 427, il est décrit un procédé d'ammoxydation du propane ou du butane, en présence d'un catalyseur pouvant comprendre notamment des oxydes de vanadium, d'antimoine et de fer ou de titane ou de chrome ou de gallium ou d'étain, ainsi qu'éventuellement d'autres métaux. Ces catalyseurs présentent la caractéristique essentielle d'avoir été calcinés à une température égale ou supérieure à 780°C.

De même, dans le brevet EP-A-0 558 424, il a été décrit un procédé d'ammoxydation d'alcanes, catalysé par des oxydes mixtes de vanadium, d'antimoine, de fer et/ou de gallium et/ou d'indium. La préparation de ces oxydes mixtes se fait par mélange de suspensions aqueuses de composés des différents métaux, chauffage sous agitation, puis évaporation de l'eau, séchage et calcination.Un tel catalyseur donne une faible sélectivité en acrylonitrile dans l'ammoxidation du propane.

Le brevet US4 257 921 décrit la fabrication d'un catalyseur d'ammoxydation d'alcanes par précipitation à partir d'une solution acide de composés des différents métaux avec neutralisation par de l'ammoniaque, <le catalyseur obtenu présente également une faible sélectivité en acrylonitrile et favorise plutôt la combustion des alcanes.

La présente invention concerne un nouveau procédé de préparation d'un oxyde mixte de vanadium, d'antimoine et de fer, répondant à la formule empirique générale (I):

V Sbₐ Fe_{b} Oₓ (I)

dans laquelle :
- a représente un nombre entier ou fractionnaire égal ou supérieur à 0,1
- b représente un nombre entier ou fractionnaire égal ou supérieur à 0,5
- x représente un nombre entier ou fractionnaire déterminé par le degré d'oxydation des autres éléments,
caractérisé en ce que :
- des composés respectifs de vanadium, d'antimoine et de fer sont mis en solution dans au moins un alcool saturé ou dans l'eau acide ayant un pH inférieur ou égal à 2,
- la solution alcoolique ou la solution aqueuse ainsi obtenue est mise en contact avec une solution aqueuse contenant un sel d'ammonium, afin de précipiter l'oxyde mixte,
- l'oxyde mixte obtenu est séparé et calciné.

Les composés du vanadium, de l'antimoine et du fer mis en oeuvre dans le procédé doivent être solubles dans un alcool saturé ou un mélange d'alcools saturés ou dans l'eau.

Dans le présent texte, on considère qu'un composé est soluble lorsque sa solubilité mesurée à 25 °C est d'au moins 5 grammes par litre d'alcool saturé ou d'eau. Ces composés peuvent être introduits ensemble ; ils peuvent aussi tout d'abord être mis séparément en solution dans un alcool, les différentes solutions alcooliques ainsi obtenues étant ensuite mélangées entre elles. Ils peuvent aussi tout d'abord être mis séparément en solution dans l'eau, les différentes solutions aqueuses ainsi obtenues étant ensuite mélangées entre elles. Généralement, mais non limitativement, on prépare une solution alcoolique ou une solution aqueuse par dissolution des différents composés, sans préparation intermédiaire de solutions de chacune des composés du vanadium, de l'antimoine et du fer.

Pour éviter la décomposition ou la précipitation de certains composés, plus particulièrement des sels de fer, on préfère utiliser une solution aqueuse acide ayant un pH inférieur ou égal à 2, lorsque la préparation des catalyseurs de l'invention est effectuée dans l'eau.

Dans ce cas l'acide utilisé pour préparer cette solution aqueuse acide peut être tout acide fort minéral ou organique susceptible de donner une solution ayant un pH inférieur ou égal à 2 et de préférence un pH de 0,5 à 1.

A titre d'exemples non limitatifs de tels acides, on peut citer notamment les acides chlorhydrique, bromhydrique, sulfurique, nitrique, trifluoroacétique.

Par commodité, on utilise de préférence l'acide correspondant au sel de fer mis en oeuvre, sans que cela soit impératif.

Souvent on utilise l'acide chlorhydrique, généralement sous forme de solutions aqueuses ayant une concentration en poids par poids de 10 à 20 %.

Dans le présent texte, l'expression solution aqueuse utilisée dans le cadre du procédé de préparation des catalyseurs couvre, sauf mention contraire, également les solutions aqueuses acides précédentes.

A titre d'exemples de composés solubles du vanadium, on peut citer l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le trichlorure de vanadium, le tétrachlorure de vanadium, le triiodure de vanadium.

A titre d'exemples de composés solubles de l'antimoine, on peut citer le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

A titre d'exemples de composés solubles du fer, on peut citer le dichlorure de fer, le trichlorure de fer, le dibromure de fer, le tribromure de fer, le diiodure de fer, le nitrate ferreux, le sulfate ferreux, le sulfate ferrique, le thiosulfate ferreux, le formiate ferrique, l'acétate ferrique, l'acétylacétonate ferrique, le benzoate ferrique, l'oléate ferrique, le lactate ferreux, le lactate ferrique.

Les alcools saturés mis en oeuvre dans le procédé de l'invention sont plus particulièrement les alcanols et les cycloalcanols. De préférence, on utilisera les alcanols et cycloalcanols dont la température d'ébullition ne soit pas trop élevée, afin de faciliter les opérations de séparation ou de recyclage par distillation ou évaporation. Ainsi les alcanols ayant de 1 à 6 atomes de carbone, tels que le méthanol, l'éthanol, le n.propanol, le propanol-2, le n.butanol, le butanol-2, le tertiobutanol, les pentanols et les hexanols, et le cyclohexanol sont préférés.

La solution alcoolique ou la solution aqueuse obtenue précédemment est ensuite mélangée à une solution aqueuse d'un sel d'ammonium, de manière à précipiter les oxydes mixtes. De préférence, on opère dans une solution aqueuse d'un carboxylate d'ammonium (par exemple acétate, citrate, tartrate), d'oxalate d'ammonium, de carbonate d'ammonium, d'hydrogénophosphate d'ammonium, permettant d'avoir un pH compris entre 5 et 9 et de préférence proche de 7. Ainsi l'acétate d'ammonium à une concentration de deux moles par litre dans l'eau présente un pH proche de 7.

Afin de maintenir la valeur du pH de la solution de préférence à une valeur proche de 7, il peut être nécessaire de neutraliser progressivement l'acidité éventuellement formée lors de la précipitation des oxydes mixtes (par exemple d'acide halohydrique formé lorsque l'on met en oeuvre un halogénure d'antimoine et/ou un halogénure de fer et/ou un halogénure de vanadium) ou l'acidité éventuelle de la solution aqueuse mise en oeuvre, à l'aide d'un composé basique. Il est préféré dans le procédé de l'invention de réaliser cette neutralisation par ajout progressif contrôlé d'ammoniaque.

Après précipitation des oxydes mixtes de l'invention, on les sépare du liquide alcool saturé/eau ou eau par toute technique habituellement utilisée pour ce type d'opération, notamment par filtration. On procède ensuite à un séchage des oxydes mixtes isolés, sous pression atmosphérique ou sous pression réduite, à une température située par exemple entre 30 °C et 200 °C, sans que ces valeurs aient une importance critique.

Les oxydes mixtes de formule (I) sont ensuite soumis à une calcination à une température de 400 °C à 800 °C. De préférence la calcination est effectuée à une température de 500 °C à 750 °C.

Parmi les oxydes mixtes de formule (I) définis précédemment, on préfère ceux pour lesquels :
- a représente un nombre entier ou fractionnaire égal ou inférieur à 100
- b représente un nombre entier ou fractionnaire égal ou inférieur à 100
- x représente un nombre entier ou fractionnaire déterminé par le degré d'oxydation des autres éléments.

Enfin on préfère encore plus particulièrement les oxydes mixtes de formule (I) pour lesquels:
- a représente un nombre entier ou fractionnaire de 0,5 à 50
- b représente un nombre entier ou fractionnaire de 1 à 50
- x représente un nombre entier ou fractionnaire déterminé par le degré d'oxydation des autres éléments.

La présente invention a également pour objet un procédé d'ammoxydation d'alcanes en phase vapeur en présence d'un catalyseur solide comportant au moins une phase active, caractérisé en ce que la dite phase active comprend au moins un oxyde mixte répondant à la formule générale empirique (I) et préparé selon le procédé défini précédemment.

Selon la présente invention des alcanes ayant de 3 à 12 atomes de carbone par molécule sont mis à réagir en phase vapeur avec de l'ammoniac et de l'oxygène en présence d'un catalyseur dont la phase active vient d'être indiquée.

Bien entendu, dans le cadre du présent procédé, on peut utiliser des gaz diluants, inertes dans les conditions réactionnelles tels que l'hélium, l'azote et l'argon. De même, de la vapeur d'eau peut être ajoutée au mélange gazeux réactionnel dans de larges limites. Ainsi le gaz réactif (alcane, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention, la teneur en gaz réactif sera d'au moins 3 % et de préférence d'au moins 20 %.

Au sein du gaz réactif les teneurs volumiques respectives en alcane, ammoniac et oxygène peuvent varier dans de larges limites.

La teneur en alcane dans le gaz réactif est de préférence comprise entre 5 et 70 %. Celle en ammoniac est de préférence comprise entre 3 et 50 % et celle en oxygène est de préférence comprise entre 3 et 45 %.

Pour une bonne mise en oeuvre du procédé selon l'invention, la composition du mélange réactif sera de préférence choisie en dehors du domaine d'explosivité.

Au départ de propane, on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile et divers autres produits intermédiaires bien connus de l'homme de l'art.

Au départ d'isobutane, on obtiendra un mélange renfermant du méthacrylonitrile et de l'iso-butène ou des n-butènes.

Le procédé selon l'invention convient plus particulièrement à l'ammoxydation du propane.

Si l'alcane mis en oeuvre peut être de qualité technique, il ne renfermera pas de quantités importantes de composés à insaturation éthylénique. Ainsi le propane engagé ne renfermera généralement que des quantités mineures de propylène, par exemple inférieures à 10 %.

Le procédé selon l'invention est réalisé sous forme de réaction en phase vapeur. En conséquence, n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 300°C et 550°C et, de préférence, entre 400°C et 500°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 h⁻¹ et, de préférence, entre 200 et 20000 h⁻¹.

La vitesse volumique horaire se définit comme le rapport volume gazeux total/volume du catalyseur/heure.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Le catalyseur utilisé dans le procédé d'ammoxydation des alcanes peut comprendre uniquement un ou plusieurs oxydes mixtes de vanadium, d'antimoine et de fer définis précédemment, constituant la phase active du catalyseur, ou comprendre en outre un oxyde minéral, tel que par exemple l'alumine, la silice, la silice-alumine, la zircone, la cérine, la magnésie, l'oxyde de titane, l'oxyde de niobium, sur lequel ladite phase active est déposée ou avec lequel la phase active est mélangée, en utilisant différentes techniques connues de l'homme du métier, telles que l'imprégnation ou le dépôt par "slurry".

La phase catalytique, constituée de la phase active seule ou de la phase active déposée sur un oxyde minéral ou mélangée avec ledit oxyde minéral, peut ensuite être mise en oeuvre sous forme massique ou à l'état particulaire ; elle peut donc être utilisée sous forme de poudre ou être mise en forme, par exemple de billes, pastilles, extrudés, particules concassées , selon différentes techniques connues.

Pour une mise en oeuvre du procédé en lit fixe, ces techniques peuvent être, par exemple, le pastillage, l'enrobage sur un substrat inerte ou sur substrat céramique ou métallique de type monolithique.

Pour une mise en oeuvre du procédé en lit mobile ou en lit fluidisé, la phase catalytique est généralement mise en forme par atomisation, séchage et calcination.

Les phases catalytiques peuvent par exemple être mises en forme par compression, de façon à obtenir des pastilles. Ces pastilles peuvent ensuite être éventuellement concassées en éclats. Les valeurs précisés de la pression, du diamètre et de l'épaisseur des pastilles, et de la granulométrie des éclats pourront être choisies par l'homme de l'art en fonction de la perte de charge admissible dans le réacteur.

Une variante de préparation de la phase catalytique peut consister à réaliser en une seule étape, la synthèse de la phase active et son dépôt sur un oxyde minéral ou son mélange avec ledit oxyde minéral.

Les phases catalytiques peuvent également être déposées sur un substrat inerte ou l'enrober. La nature de ce substrat n'est pas critique dès lors qu'il est chimiquement inerte vis-à-vis des réactifs dans les conditions réactionnelles choisies. A titre d'exemples de substrats susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce substrat est de préférence non poreux et peut notamment être à base d'oxyde réfractaire sous forme particulaire, le substrat le plus couramment employé étant à base d'argile. Ce substrat peut par exemple être constitué de billes d'argile, inertes, pleines, solides et rugueuses, de diamètre compris entre 0.5 et 6 mm. La valeur précise du diamètre des billes pourra être choisie en fonction de la perte de charge admissible dans le réacteur. Ce substrat peut également être rendu non poreux par émaillage.

Ce substrat peut également être un substrat céramique, ledit substrat étant de préférence sous forme d'une structure inerte et rigide de type monolithique comprenant des canaux ou conduits. De tels supports sont bien connus et ont été largement décrits dans la littérature. Les substrats en matières céramiques utilisés sont notamment ceux comprenant comme matière principale la cordiérite, l'alumine, la mullite, la porcelaine, les carbures de bore ou de silicium.

Ce substrat peut également être un substrat métallique. De tels substrats sont bien connus. Les substrats métalliques convenables sont notamment ceux obtenus à partir d'alliages de fer, de nickel et de chrome, ou ceux obtenus à partir d'alliages de fer, de chrome, aluminium et cobalt tels que ceux connus sous la marque KANTHAL ou ceux obtenus à partir d'alliages de fer, de chrome, d'aluminium et d'yttrium connus sous la marque FECRALLOY. Le métal peut aussi être de l'acier carboné ou de la fonte simple.

Lorsqu'on fait appel à un catalyseur enrobé, la quantité de phase catalytique qui peut varier dans de larges limites, est en pratique comprise entre 1 et 50 % et de préférence entre 5 et 35 % en poids par rapport à l'ensemble substrat + phase catalytique.

Ainsi, certains catalyseurs, utiles pour une mise en oeuvre du procédé en lit fixe, peuvent être obtenus par enrobage de manière connue en soi, des phases catalytiques, intermédiaires ou finies, broyées. Cette méthode classique consiste à déposer autour de billes inertes, mais rugueuses, une couche de phase catalytique intermédiaire ou finie. Une fois les billes recouvertes de la quantité voulue de la phase catalytique, elles sont séchées par de l'air chaud, entre 70 et 150°C pendant au moins 30 minutes, puis introduites dans un four pour être calcinées entre 300 et 600°C de préférence entre 450 et 500°C, pendant au moins 3 heures.

Certains catalyseurs utiles pour une mise en oeuvre du procédé selon l'invention en lit mobile ou en lit fluidisé peuvent être obtenus par la technique connue en soi du séchage par atomisation en atmosphère, de préférence non réductrice. Par une telle opération, le cas échéant suivie d'une calcination à une température de l'ordre de 400 à 800°C, on obtient des poudres de forme sphérique de diamètre compris entre 5 et 700 µm. Des poudres constituées pour au moins 80 % en poids de particules dont la dimension est comprise entre 5 et 200 µm sont préférées dans le cadre d'une utilisation en lit fluidisé.

La phase catalytique, seule ou ainsi mise en oeuvre sous forme massique ou à l'état particulaire, constitue le catalyseur.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti, du propylène, des hydrocarbures légers et le cas échéant du CO₂. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

### EXEMPLES DE PREPARATION D'OXYDES MIXTES.

### EXEMPLE 1 - Préparation de l'oxyde mixte (A) selon l'invention, de formule empirique suivante :V Sb5 Fe2,5 Ox

On prépare une solution de 7,77 g de FeCl₃,6H₂O, de 3,05 g d'acétylacétonate de vanadyle et de 17,22 g de SbCl₅ dans 100 ml d'une solution aqueuse d'acide chlorhydrique ayant une concentration de 15 % en poids par poids.

La solution aqueuse est versée goutte à goutte dans 500 ml de solution aqueuse contenant de l'acétate d'ammonium (2 moles/litre) afin d'avoir un pH initial de l'ordre de 7,0. Pendant la précipitation des oxydes mixtes de V, Sb et Fe, le pH qui tend à diminuer en raison de la libération d'acide chorhydrique et de la présence d'acide chlorhydrique dans la solution aqueuse, est maintenu constant par addition d'une solution concentrée d'ammoniaque (à 30 % en poids).

Le précipité ainsi formé est isolé par filtration, lavé à l'eau, séché pendant 12 h à 140 °C, puis il est calciné pendant 3 h à 700 °C.

Le composé de formule V Sb₅ Fe_{2,5} Oₓ ainsi obtenu est ensuite comprimé sous une pression de 4300 bars. On obtient alors des pastilles de 3 cm de diamètre et d'environ 0,5 cm d'épaisseur. Ces pastilles sont concassées en éclats de granulométrie comprise entre 0,3 et 0,8 cm constituant le catalyseur (A) conforme à l'invention.

### EXEMPLES 2 A 7 - Préparation de différents oxvdes mixtes selon l'invention.

On prépare différents oxydes mixtes, en suivant le mode opératoire décrit dans l'exemple 1, mais en utilisant des quantités différentes d'acétylacétonate de vanadyle (exemples 2 et 5) ou de pentachlorure d'antimoine et de chlorure de ferrique (exemples 3, 4, 6 et 7).

On obtient ainsi les oxydes mixtes selon l'invention présentant les formules suivantes :

| | |
|---|---|
| exemple 2 | VSb₁₀Fe₅Oₓ (référence B) |
| exemple 3 | VSb_{12,5}Fe₅Oₓ (référence C) |
| exemple 4 | VSb_{17,5}Fe₅Oₓ (référence D) |
| exemple 5 | VSb₂₀Fe₁₀Oₓ (référence E) |
| exemple 6 | VSb₂₅Fe₁₀Oₓ (référence F) |
| exemple 7 | VSb₃₀Fe₁₀Oₓ (référence G) |

### EXEMPLES 8 ET 9 - Préparation des oxydes mixtes (H) et (J) selon l'invention

On prépare une solution de 7,77 g de FeCl₃,6H₂O, de 1,52 g d'acétylacétonate de vanadyle et de 21,4 g de SbCl₅ dans 100 ml d'éthanol absolu.

La solution éthanolique est versée goutte à goutte dans 500 ml de solution aqueuse contenant de l'acétate d'ammonium (2 moles/litre) afin d'avoir un pH initial de l'ordre de 7,0. Pendant la précipitation des oxydes mixtes de V, Sb et Fe, le pH qui tend à diminuer en raison de la libération d'acide chorhydrique, est maintenu constant par addition d'une solution concentrée d'ammoniaque (à 30 % en poids).

Le précipité ainsi formé est isolé par filtration, lavé à l'eau, séché pendant 12 h à 140 °C, puis il est calciné pendant 3 h à 700 °C.

Le composé de formule V Sb_{12,5} Fe₅ Oₓ ainsi obtenu est ensuite comprimé sous une pression de 4300 bars. On obtient alors des pastilles de 3 cm de diamètre et d'environ 0,5 cm d'épaisseur. Ces pastilles sont concassées en éclats de granulométrie comprise entre 0,3 et 0,8 cm constituant le catalyseur (H) selon l'invention.

On prépare selon le mode opératoire utilisé pour le catalyseur (H), mais en utilisant des quantités différentes de chlorure ferrique et de pentachlorure d'antimoine, un catalyseur (J) selon l'invention de composition V Sb₂₀ Fe₁₀ Oₓ.

### MODE OPERATOIRE GENERAL DES TESTS D'AMMOXYDATION

L'échantillon de catalyseur est préalablement porté sur un banc de mesure à la température de 150°C sous balayage d'hélium pendant 10 min, puis il est soumis à un flux gazeux dont la composition sera précisée pour chaque exemple et qui renferme du propane, de l'ammoniac, de l'oxygène, de l'hélium et le cas échéant de la vapeur d'eau.

La pression totale du mélange réactionnel, comprise entre 1 et 6 bar, sera également précisée pour chaque exemple.

Le débit total gazeux est défini de façon à avoir une vitesse volumique horaire (VVH) comprise entre 100 et 36000 h⁻¹, dont la valeur précise sera indiquée pour chaque exemple.

Volume de phase active : 25 cm³.

Le principe du test d'ammoxydation du propane est le suivant :
- On porte le catalyseur à une température T₁, par exemple 310°C, et après 30 min de stabilisation à la température T₁, on détermine par chromatographie en phase gazeuse la composition du mélange en sortie de réacteur.
- On calcule les pourcentages de conversion et les sélectivités obtenus sur le catalyseur examiné à la température d'entrée T₁ par des relations du type:
   conversion du propane (en moles %) = propane transformé/propane introduit
   sélectivité en acrylonitrile (en moles %) = propane transformé en acryionitrile/propane converti.
- On porte ensuite le catalyseur de 310 à 550°C par incrément de 20°C et on détermine toutes les 40 min les pourcentages de conversion et les sélectivités.

Dans les exemples d'ammoxydation ci-après, les conventions suivantes sont utilisées :
Temp = température de la réaction
tc = temps de contact
TTC₃H₈ = conversion du propane
SACN = sélectivité en acrylonitrile
SACN+C₃H₆ = sélectivité en acrylonitrile et propylène
SAMMOX = sélectivité en acétonitrile, en acide cyanhydrique et en autres sous-produits d'ammoxydation
Prod ACN = productivité en acrylonitrile exprimée en g d'acrylonitrile formé/litre de catalyseur x heure.
Le complément à 100 % des sélectivités correspond à la formation de CO et de CO₂ ainsi qu'éventuellement de méthane, d'éthane et d'éthylène.

### EXEMPLES D'AMMOXYDATION DU PROPANE

### EXEMPLES 10 A 20

On réalise l'ammoxydation du propane, comme décrit précédemment, en utilisant comme catalyseurs les phases actives constituées par les oxydes mixtes préparés selon l'invention A, B, C, D, E, F, G, H et J.

Les conditions particulières mises en oeuvre sont les suivantes :
- vitesse volumique horaire = 1800 h⁻¹ ou 3600 h⁻¹ ou 7200 h⁻¹
- pression totale = 1,3 bar
- composition volumique du mélange réactionnel : C₃H₈ = 25 %
   NH₃ = 10 %
   O₂ = 20 %
   He = 45 %

Les conditions de température et les résultats obtenus sont rassemblés dans le tableau 1 ci-après.

**Tableau 1**

| Exemples | Oxyde mixte utilisé | Temp (°C) | tc | VVH (h⁻¹) | TT C3H8 (en %) | SACN (en %) | SACN + C3H6 (en %) | SAMMOX (en %) | Prod ACN (g/l x h) |
|---|---|---|---|---|---|---|---|---|---|
| Exemple 10 | | 365 | | | 12 | 17 | 40 | 21 | 20 |
| | A | 385 | 2 s | 1800 | 24 | 17 | 33 | 13 | 40 |
| | | 405 | | | 27 | 16 | 37 | 14 | 43 |
| | | 425 | | | 29 | 18 | 41 | 14 | 52 |
| Exemple 11 | | 390 | | | 10 | 27 | 35 | 31 | 27 |
| | B | 415 | 2 s | 1800 | 29 | 26 | 31 | 11 | 75 |
| | | 430 | | | 29 | 34 | 39 | 10 | 98 |
| | | 445 | | | 29 | 35 | 41 | 8 | 101 |
| Exemple 12 | C | 410 | 2 s | 1800 | 15 | 35 | 39 | 14 | 52 |
| | | 430 | | | 16 | 46 | 49 | 11 | 73 |
| Exemple 13 | C | 410 | 1 s | 3600 | 9 | 47 | 51 | 18 | 84 |
| | | 430 | | | 12 | 49 | 53 | 13 | 117 |
| Exemple 14 | | 410 | | | 3 | 53 | 68 | 21 | 63 |
| | C | 430 | 0,5s | 7200 | 7 | 54 | 62 | 25 | 150 |
| | | 450 | | | 11 | 60 | 69 | 17 | 262 |
| | | 470 | | | 15 | 53 | 58 | 12 | 316 |
| Exemple 15 | | 410 | | | 5 | 41 | 50 | 23 | 20 |
| | D | 430 | 2 s | 1800 | 6 | 43 | 50 | 16 | 26 |
| | | 450 | | | 8 | 48 | 55 | 13 | 38 |
| | | 470 | | | 11 | 51 | 59 | 8 | 56 |
| Exemple 16 | | 410 | | | 8 | 35 | 42 | 12 | 28 |
| | E | 430 | 2 s | 1800 | 10 | 39 | 45 | 10 | 39 |
| | | 450 | | | 18 | 44 | 49 | 8 | 79 |
| | | 470 | | | 26 | 40 | 44 | 7 | 103 |
| Exemple 17 | | 410 | | | 4 | 42 | 46 | 35 | 17 |
| | F | 430 | 2 s | 1800 | 8 | 49 | 52 | 21 | 39 |
| | | 450 | | | 12 | 50 | 53 | 14 | 60 |
| | | 470 | | | 15 | 47 | 49 | 12 | 70 |
| Exemple 18 | | 410 | | | 3 | 56 | 59 | 25 | 17 |
| | G | 430 | 2 s | 1800 | 5 | 55 | 57 | 25 | 27 |
| | | 450 | | | 8 | 60 | 62 | 18 | 48 |
| | | 470 | | | 10 | 60 | 61 | 15 | 60 |
| Exemple 19 | | 410 | | | 6 | 39 | 45 | 30 | 23 |
| | H | 430 | 2 s | 1800 | 8 | 47 | 52 | 23 | 37 |
| | | 450 | | | 13 | 49 | 54 | 16 | 63 |
| | | 470 | | | 15 | 48 | 53 | 12 | 71 |
| Exemple 20 | | 410 | | | 6 | 33 | 40 | 24 | 20 |
| | J | 430 | 2 s | 1800 | 9 | 40 | 44 | 20 | 36 |
| | | 450 | | | 14 | 49 | 53 | 13 | 68 |
| | | 470 | | | 20 | 46 | 49 | 8 | 91 |

## Revendications

1. Procédé de préparation d'un oxyde mixte comportant du vanadium, de l'antimoine et du fer, répondant à la formule empirique générale (I) :
V Sbₐ Fe_{b} Oₓ (I)
dans laquelle :
- a représente un nombre entier ou fractionnaire égal ou supérieur à 0,1
- b représente un nombre entier ou fractionnaire égal ou supérieur à 0,5
- x représente un nombre entier ou fractionnaire déterminé par le degré d'oxydation des autres éléments,
**caractérisé en ce que** :
- des composés respectifs de vanadium, d'antimoine et de fer sont mis en solution dans au moins un alcool saturé,
- la solution alcoolique ainsi obtenue est mise en contact avec une solution aqueuse contenant un sel d'ammonium, afin de précipiter l'oxyde mixte,
- l'oxyde mixte obtenu est séparé et calciné.

2. Procédé de préparation d'un oxyde mixte comportant du vanadium, de l'antimoine et du fer, répondant à la formule empirique générale (I) :
V Sbₐ Fe_{b} Oₓ (I)
dans laquelle :
- a représente un nombre entier ou fractionnaire égal ou supérieur à 0,1
- b représente un nombre entier ou fractionnaire égal ou supérieur à 0,5
- x représente un nombre entier ou fractionnaire déterminé par le degré d'oxydation des autres éléments,
**caractérisé en ce que** :
- des composés respectifs de vanadium, d'antimoine et de fer sont mis en solution dans une solution aqueuse acide ayant un pH inférieur ou égal à 2,
- la solution aqueuse ainsi obtenue est mise en contact avec une solution aqueuse contenant un sel d'ammonium, afin de précipiter l'oxyde mixte,
- l'oxyde mixte obtenu est séparé et calciné.

3. Procédé selon la revendication 2, **caractérisé en ce que** l'acide utilisé pour préparer la solution aqueuse acide est tout acide fort minéral ou organique susceptible de donner une solution ayant un pH inférieur ou égal à 2 et de préférence un pH de 0,5 à 1.

4. Procédé selon l'une des revendications 2 ou 3, **caractérisé en ce que** l'acide utilisé pour préparer la solution aqueuse acide est choisi parmi les acides chlorhydrique, bromhydrique, sulfurique, nitrique, trifluoroacétique.

5. Procédé selon l'une des revendications 1 à 4, **caractérisé en ce que** le composé soluble du vanadium est choisi parmi l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le trichlorure de vanadium, le tétrachlorure de vanadium, le triiodure de vanadium.

6. Procédé selon l'une des revendications 1 à 5, **caractérisé en ce que** le composé soluble de l'antimoine est choisi parmi le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

7. Procédé selon l'une des revendications 1 à 6, **caractérisé en ce que** le composé soluble du fer est choisi parmi le dichlorure de fer, le trichlorure de fer, le dibromure de fer, le tribromure de fer, le diiodure de fer, le nitrate ferreux, le sulfate ferreux, le sulfate ferrique, le thiosulfate ferreux, le formiate ferrique, l'acétate ferrique, l'acétylacétonate ferrique, le benzoate ferrique, l'oléate ferrique, le lactate ferreux, le lactate ferrique.

8. Procédé selon l'une des revendications 1, 5, 6 ou 7, **caractérisé en ce que** les alcools saturés mis en oeuvre sont les alcanols et les cycloalcanols et de préférence les alcanols ayant de 1 à 6 atomes de carbone et le cyclohexanol.

9. Procédé selon l'une des revendications 1 à 8, **caractérisé en ce que** la solution alcoolique ou la solution aqueuse obtenue est ensuite mélangée à une solution aqueuse d'un sel d'ammonium, de manière à précipiter les oxydes mixtes, de préférence à une solution aqueuse d'un carboxylate d'ammonium, d'oxalate d'ammonium, de carbonate d'ammonium, d'hydrogénophosphate d'ammonium, permettant d'avoir un pH compris entre 5 et 9 et de préférence proche de 7.

10. Procédé selon la revendication 9, **caractérisé en ce que** l'on maintient la valeur du pH de la solution à une valeur proche de 7, en neutralisant progressivement l'acidité formée lors de la précipitation des oxydes mixtes ou provenant de la solution aqueuse mise en oeuvre, à l'aide d'un composé basique, par ajout progressif contrôlé d'ammoniaque.

11. Procédé selon l'une des revendications 1 à 10, **caractérisé en ce que** après séparation des oxydes mixtes, ceux-ci sont soumis à une calcination à une température de 400 °C à 800 °C.

12. Procédé selon l'une des revendications 1 à 11, **caractérisé en ce qu'**il est appliqué à la préparation d'oxydes mixtes de formule générale (I) dans laquelle :
- a représente un nombre entier ou fractionnaire égal ou inférieur à 100 et de préférence un nombre entier ou fractionnaire de 0,5 à 50
- b représente un nombre entier ou fractionnaire égal ou inférieur à 100 et de préférence un nombre entier ou fractionnaire de 1 à 50
- x représente un nombre entier ou fractionnaire déterminé par le degré d'oxydation des autres éléments.

13. Utilisation d'un catalyseur solide obtenu par un procédé de fabrication selon l'une des revendications 1 à 12 dans un procédé d'ammoxydation d'alcanes en phase vapeur.

14. Utilisation selon la revendication 13, **caractérisé en ce que** les alcanes comprenant de 3 à 12 atomes de carbone par molécule, choisis parmi le propane et l'isobutane et sont mis à réagir en phase vapeur avec de l'ammoniac et de l'oxygène.

15. Utilisation selon l'une des revendications 13 ou 14, **caractérisé en ce que** au sein du gaz réactif, constitué par l'alcane, l'ammoniac, l'oxygène, la teneur en alcane est comprise entre 5 et 70 %, la teneur en ammoniac est comprise entre 3 et 50 % et la teneur en oxygène est comprise entre 3 et 45 %.

16. Utilisation selon l'une des revendications 13 à 15, **caractérisé en ce que** la température de réaction est comprise entre 300°C et 550°C.

## Patentansprüche

1. Verfahren zur Herstellung eines gemischten Oxides, umfassend Vanadium, Antimon und Eisen, das der folgenden empirischen Formel entspricht
VSbₐFe_{b}Oₓ (I)
in der
- a eine ganze oder gebrochene Zahl von gleich oder höher 0,1 darstellt,
- b eine ganze oder gebrochene Zahl von gleich oder höher 0,5 darstellt,
- x eine ganze oder gebrochene Zahl darstellt, die durch den Oxidationsgrad der anderen Elemente bestimmt wird,
**dadurch gekennzeichnet, daß**
- die Verbindungen von jeweils Vanadium, Antimon und Eisen in Lösung in mindestens einem gesättigten Alkohol gebracht werden,
- die auf diese weise erhaltene alkoholische Lösung in Kontakt mit einer wäßrigen Lösung gebracht wird, die ein Ammoniumsalz enthält, um das gemischte Oxid auszufällen,
- das erhaltene gemischte Oxid abgetrennt und kalziniert wird.

2. Verfahren zur Herstellung eine gemischten Oxides, umfassend Vanadium, Antimon und Eisen, das der folgenden empirischen Formel entspricht
VSbₐFe_{b}Oₓ (I)
in der
- a eine ganze oder gebrochene Zahl von gleich oder höher 0,1 daratellt,
- b eine ganze oder gebrochene Zahl von gleich oder höher 0,5 darstellt,
- x eine ganze oder gebrochene Zahl darstellt, die durch den Oxidationsgrad der anderen Elemente bestimmt wird,
**dadurch gekennzeichnet, daß**
- die Verbindungen von jeweils Vanadium, Antimon und Eisen in Lösung in einer wäßrigen, sauren Lösung mit einem pH-Wert von unterhalb oder gleich 2 gebracht werden,
- die auf diese Weise erhaltene wäßrige Lösung in Kontakt mit einer wäßrigen Lösung gebracht wird, die ein Ammoniumsalz enthält, um das gemischte Oxid auszufällen,
- das erhaltene gemischte Oxid abgetrennt und kalziniert wird.

3. Verfahren nach Anspruch 2, **dadurch gekennzeichnet, daß** die für die Herstellung der wäßrigen, sauren Lösung verwendete Säure jede starke Mineralsäure oder organische Säure ist, die fähig ist, eine Lösung mit einem pH-Wert von unterhalb oder gleich 2 und vorzugsweise einem pH-Wert von 0,5 bis 1 zu ergeben.

4. Verfahren nach einem der Ansprüche 2 oder 3, **dadurch gekennzeichnet, daß** die für die Herstellung der wäßrigen, sauren Lösung verwendete Säure unter Chlorwasserstoffsäure, Bromwasserstoffsäure, Schwefelsäure, Salpetersäure, Trifluoressigsäure ausgewählt wird.

5. Verfahren nach einem der Ansprüche 1 bis 4, **dadurch gekennzeichnet, daß** die lösliche Verbindung des Vanadiums unter Vanadylacetylacetonat, Vanadyltrichlorid, Vanadiumtrifluorid, Vanadiumtecrafluorid, Vanadiumpentafluorid, Vanadiumtribromid, Vanadiumdichlorid, Vanadiumtrichlorid, Vanadiumtetrachlorid, Vanadiumtriiodid ausgewählt wird.

6. Verfahren nach einem der Ansprüche 1 bis 5, **dadurch gekennzeichnet, daß** die lösliche Verbindung des Antimons unter Antimonpentachlorid, Antimontrichlorid, Antimontribromid, Antimontrifluorid, Antimontriiodid, Antimontrioxid, Antimonhydrid ausgewählt wird.

7. Verfahren nach einem der Ansprüche 1 bis 6, **dadurch gekennzeichnet, daß** die lösliche Verbindung des Eisens unter Eisendichlorid, Eisentrichlorid, Eisendibromid, Eisentribromid, Eisendiiodid, Eisen(II)-nitrat, Eisen(II)-sulfat, Eisen(III)-sulfat, Eisen(II)-thiosulfat, Eisen(III)-formiat, Eisen(III)-acetat, Eisen (III) -acetylacetonat, Eisen(III)-benzoat, Eisen(III)-oleat, Eisen(II)-lactat, Eisen(III)-lactat ausgewählt wird.

8. Verfahren nach einem der Ansprüche 1, 5, 6 oder 7, **dadurch gekennzeichnet, daß** die eingesetzten gesättigten Alkohole Alkanole und Cycloalkanole sind, vorzugsweise Alkanole mit 1 bis 6 Kohlenstoffatomen und Cyclohexanol.

9. Verfahren nach einem der Ansprüche 1 bis 8, **dadurch gekennzeichnet, daß** die erhaltene alkoholische oder wäßrige Lösung anschließend mit einer wäßrigen Lösung eines Ammoniumsalzes vermischt wird, so daß die gemischten Oxide ausfallen, vorzugsweise mit einer wäßrigen Lösung eines Ammoniumcarboxylates, von Ammoniumoxalat, Ammoniumcarbonat, Ammoniumhydrogenphosphat, die ermöglichen, einen pH-Wert zwischen 5 und 9, vorzugsweise in der Nähe von 7 zu erhalten.

10. Verfahren nach Anspruch 9, **dadurch gekennzeichnet, daß** man den pH-Wert der Lösung auf einem Wert von nahe 7 hält, indem man schrittweise die bei der Fällung des gemischten Oxides gebildete oder von der eingesetzten wäßrigen Lösung stammende Azidität mit Hilfe einer basischen Verbindung, durch schrittweise kontrollierte Zugabe von Ammoniak, neutralisiert.

11. Verfahren nach einem der Ansprüche 1 bis 10, **dadurch gekennzeichnet, daß** die gemischten Oxide nach ihrer Abtrennung einer Kalzinierung bei einer Temperatur von 400 °C bis 800 °C unterzogen werden.

12. Verfahren nach einem der Ansprüche 1 bis 11, **dadurch gekennzeichnet, daß** es bei der Herstellung von gemischten Oxiden der allgemeinen Formel (I) angewendet wird, in der
- a eine ganze oder gebrochene Zahl von gleich oder unter 100 und vorzugsweise eine ganze oder gebrochene Zahl von 0,5 bis 50 darstellt,
- b eine ganze oder gebrochene Zahl von gleich oder unter 100 und vorzugsweise eine ganze oder gebrochene Zahl von 1 bis 50 darstellt,
- x eine ganze oder gebrochene Zahl darstellt, die durch den Oxidationsgrad der anderen Elemente bestimmt wird.

13. Verwendung eines festen Katalysators, erhalten durch ein Verfahren zur Herstellung nach einem der Ansprüche 1 bis 12, in einem Verfahren zur Ammoxidation von Alkanen in der Dampfphase.

14. Verwendung nach Anspruch 13, **dadurch gekennzeichnet, daß** die Alkane, die 3 bis 12 Kohlenstoffatome pro Molekül umfassen, unter Propan und Isobutan ausgewählt und in der Dampfphase mit Ammoniak und Sauerstoff zur Reaktion gebracht werden.

15. Verwendung nach einem der Ansprüche 13 oder 14, **dadurch gekennzeichnet, daß** in dem reaktiven Gas, das aus Alkan, Ammoniak, und Sauerstoff besteht, der Gehalt an Alkan zwischen 5 % und 70 %, der Gehalt an Ammoniak zwischen 3 % und 50 % und der Gehalt an Sauerstoff zwischen 3 % und 45 % betragen.

16. Verwendung nach einem der Ansprüche 13 bis 15, **dadurch gekennzeichnet, daß** die Temperatur der Reaktion zwischen 300 °C und 550 °C liegt.

## Claims

1. Process for the preparation of a mixed oxide containing vanadium, antimony and iron corresponding to the general empirical formula (I):
VSbₐFe_{b}Oₓ (I)
in which:
- a represents a whole or fractional number equal to or greater than 0.1
- b represents a whole or fractional number equal to or greater than 0.5
- x represents a whole or fractional number determined by the oxidation state of the other elements,
**characterized in that**;
- respective vanadium, antimony and iron compounds are dissolved in at least one saturated alcohol,
- the alcoholic solution thus obtained is brought into contact with an aqueous solution containing an ammonium salt, in order to precipitate the mixed oxide,
- the mixed oxide obtained is separated and calcined.

2. Process for the preparation of a mixed oxide containing vanadium, antimony and iron corresponding to the general empirical formula (I):
VSbₐFe_{b}Oₓ (I)
in which:
- a represents a whole or fractional number equal to or greater than 0.1
- b represents a whole or fractional number equal to or greater than 0.5
- x represents a whole or fractional number determined by the oxidation state of the other elements,
**characterized in that**:
- respective vanadium, antimony and iron compounds are dissolved in an acidic aqueous solution having a pH of less than or equal to 2,
- the aqueous solution thus obtained is brought into contact with an aqueous solution containing an ammonium salt, in order to precipitate the mixed oxide,
- the mixed oxide obtained is separated and calcined.

3. Process according to claim 2, **characterized in that** the acid used to prepare the acidic aqueous solution is any strong inorganic or organic acid capable of giving a solution having a pH of less than or equal to 2 and preferably a pH of 0.5 to 1.

4. Process according to either of claims 2 and 3, **characterized in that** the acid used to prepare the acidic aqueous solution is chosen from hydrochloric, hydrobromic, sulphuric, nitric and trifluoroacetic acids.

5. Process according to one of claims 1 to 4, **characterized in that** the soluble vanadium compound is chosen from vanadyl acetylacetonate, vanadyl trichloride, vanadium trifluoride, vanadium tetrafluoride, vanadium pentafluoride, vanadium tribromide, vanadium dichloride, vanadium trichloride, vanadium tetrachloride or vanadium triiodide.

6. Process according to one of claims 1 to 5, **characterized in that** the soluble antimony compound is chosen from antimony pentachloride, antimony trichloride, antimony tribromide, antimony trifluoride, antimony triiodide, antimony trioxide or stibine.

7. Process according to one of claims 1 to 6, **characterized in that** the soluble iron compound is chosen from iron dichloride, iron trichloride, iron dibromide, iron tribromide, iron diiodide, ferrous nitrate, ferrous sulphate, ferric sulphate, ferrous thiosulphate, ferric formate, ferric acetate, ferric acetylacetonate, ferric benzoate, ferric oleate, ferrous lactate or ferric lactate.

8. Process according to one of claims 1,5,6 and 7, **characterized in that** the saturated alcohols used are alkanols and cycloalkanols and preferably alkanols having from 1 to 6 carbon atoms and cyclohexanol.

9. Process according to one of claims 1 to 8, **characterized in that** the alcoholic solution or the aqueous solution obtained is then mixed with an aqueous solution of an ammonium salt, so as to precipitate the mixed oxides, preferably with an aqueous solution of an ammonium carboxylate. of ammonium oxalate, of ammonium carbonate or of ammonium hydrogenphosphate, which makes it possible to have a pH of between 5 and 9 and preferably in the region of 7.

10. Process according to claim 9, **characterized in that** the value of the pH of the solution is maintained at a value in the region of 7, by progressively neutralizing the acidity formed during the precipitation of the mixed oxides or which arises from the aqueous solution used, with the aid of a basic compound, by controlled progressive addition of aqueous ammonia.

11. Process according to one of claims 1 to 10, **characterized in that**, after separation of the mixed oxides, the latter are calcined at a temperature of 400°C to 800°C.

12. Process according to one of claims 1 to 11, **characterized in that** it is applied to the preparation of mixed oxides of general formula (I) in which:
- a represents a whole or fractional number equal to or less than 100 and preferably a whole or fractional number from 0.5 to 50
- b represents a whole or fractional number equal to or less than 100 and preferably a whole or fractional number from 1 to 50
- x represents a whole or fractional number determined by the oxidation state of the other elements.

13. Use of a solid catalyst obtained by a manufacturing process according to one of claims 1 to 12 in a process for the ammoxidation of alkanes in the vapour phase.

14. Use according to claim 13, **characterized in that** the alkanes comprising from 3 to 12 carbon atoms per molecule, chosen from propane and isobutane and are reacted in the vapour phase with ammonia and oxygen.

15. Use according to either of claims 13 and 14, **characterized in that** within the reactive gas, composed of alkane, ammonia and oxygen, the alkane content is between 5 and 70 %, the ammonia content is between 3 and 50 % and the oxygen content is between 3 and 45 %.

16. Use according to one of claims 13 to 15, **characterized in that** the reaction temperature is between 300°C and 550°C.
